## Europäisches Patentamt
## European Patent Office
## Office européen des brevets

(19)

(11) Numéro de publication: **0 287 441**
**A1**

(12) **DEMANDE DE BREVET EUROPEEN**

(21) Numéro de dépôt: **88400850.9**

(22) Date de dépôt: **08.04.88**

(51) Int. Cl.⁴: **A 61 F 5/44**

(30) Priorité: **17.04.87 FR 8705554**

(43) Date de publication de la demande:
**19.10.88 Bulletin 88/42**

(84) Etats contractants désignés:
**AT BE CH DE ES FR GB GR IT LI LU NL SE**

(71) Demandeur: **Nigay, Pierre**
**10 rue de Paris**
**F-60200 Compiegne (FR)**

(72) Inventeur: **Nigay, Pierre**
**10 rue de Paris**
**F-60200 Compiegne (FR)**

(74) Mandataire: **Bertrand, Didier et al**
**Cabinet Beau de Loménie 55, rue d'Amsterdam**
**F-75008 Paris (FR)**

(54) **Ensemble pour incontinent.**

㊼ Le boîtier contenant la pompe, le moteur, la batterie et le circuit électrique est parallélépipédique et comporte un corps (1) et un couvercle, le corps (1) comportant un fond rectangulaire (2) et des parois latérales perpendiculaires (3-6) entourant la périphérie du fond de manière à fermer les côtés du boîtier, à l'exception de deux zones situées en regard l'une de l'autre sur deux côtés opposés (5,6), une paroi intermédiaire perpendiculaire (7) étant prévue entre ces deux zones parallèlement auxdits côtés opposés (5,6) ; le couvercle se compose d'une plaque rectangulaire munie sur deux bords de deux parois latérales perpendiculaires correspondant auxdites zones lorsque le couvercle est monté sur le corps (1) ; des moyens de montage sont prévus pour fixer la pompe (29) et son moteur (28) respectivement de part et d'autre de ladite paroi intermédiaire (7), avec leurs axes perpendiculaires à cette paroi, ladite paroi (7) comportant un orifice (27) pour le passage de la transmission de mouvement entre le moteur (28) et la pompe (29).

Fig. 1

EP 0 287 441 A1

## Description

## Ensemble pour incontinent

L'invention concerne les sous-vêtements pour personnes atteintes d'incontinence d'urine, du type défini dans le document FR-A-2 555 044, dont on incorpore ici l'enseignement par référence.

Avant ce document, il existait des fournitures dont le rôle était d'absorber les urines, par capillarité, et de les emmagasiner jusqu'à un point de rétention plus ou moins important suivant la texture et la nature des matières premières employées.

On connaissait aussi par le document FR-A-2 498 447 une culotte de miction pour femme comportant une zone de puisage à laquelle peut s'adapter un tube souple d'évacuation relié à une poche de retenue au travers d'une pompe. Cette culotte est uniquement destinée à une évacuation volontaire : elle nécessite la commande d'un interrupteur. On imagine mal d'exiger des énurétiques nocturnes qu'ils se réveillent d'abord pour commuter l'interrupteur. De plus, il est clair que cette culotte n'est pas conçue pour que les accessoires (tuyaux, poches) soient portés en permanence par le sujet lui-même : en fin d'opération le tuyau d'évacuation doit être débranché. Cette culotte ne saurait donc répondre aux besoins des incontinents.

On connaissait par ailleurs, par le document FR-A-2 477 881, un appareil destiné aux grabataires mais non aux incontinents qui désirent mener une vie normale. L'appareil proposé n'est pas autonome : il nécessite le branchement électrique sur le secteur. Il ne permet pas d'être porté en permanence, mais nécessite d'être appliqué au moment voulu, ce qui ne convient pas aux incontinents pour lesquels ce moment n'est connu que quand il est trop tard. Finalement, cette appareil est également conçu pour une évacuation volontaire.

L'invention du document FR-A-2 555 044 avait pour objet un ensemble qui rende possible le contrôle du flux d'urines dès son commencement. Cet ensemble devait être simple et peu coûteux, sans risque pour l'utilisateur, entrant dans la catégorie de ce qu'il est convenu d'appeler : "sous-vêtements d'hygiène d'utilité courante". Surtout il s'agissait de présenter un ensemble conçu pour être porté intégralement et en permanence par le sujet, et doté d'un fonctionnement automatique, qui le rende parfaitement adapté à tous les cas d'incontinence.

L'objet du document FR-A-2 555 044 consiste donc en une sorte de culotte, slip, suspensoir dont la partie en contact avec les voies urinaires extérieures,lorsqu'il est porté, est équipée d'un système de détection d'humidité et d'un orifice d'évacuation relié à un système de pompage. Plus précisément, la culotte réalisée, au moins pour sa partie basse, en matière imperméable s'adaptant de manière étanche au porteur, comporte en partie basse une zone de puisage à laquelle s'adapte un tube souple d'évacuation relié à une poche de retenue au travers d'une pompe. L'ensemble comprend également un support porté par le sujet et retenant d'une part un boîtier logeant la pompe et une source de courant continu basse-tension, et d'autre part la poche de retenue. D'autre part, il est prévu un circuit électrique temporisé de déclenchement de la pompe, alimenté par la source de courant et comportant des moyens de déclenchement réagissant au liquide disposés près de la zone de puisage et reliés par des fils électriques souples audit dispositif.

L'ensemble de l'énergie nécessaire au fonctionnement pour la détection, la commande de mise en route et d'arrêt du système de pompage provient de piles ou batteries.

Le système de détection d'arrivée du liquide est constitué d'électrodes qui transmettent l'information à un appareillage à commande électrique.

La pompe est de préférence une pompe silencieuse auto-amorçante dont le débit est avantageusement compris entre 1 et 40 cm$^3$ à la seconde, étant entendu qu'il est de préférence voisin de 10 à 30 cm$^3$. Un débit de 900ml/min est généralement conforme aux normes en la matière.

La présente invention a pour but de perfectionner l'invention du document FR-A-2 555 044.

Un premier perfectionnement concerne la réalisation du boîtier renfermant la pompe et la source de courant continu basse- tension. Dans la mesure où l'appareil décrit dans le document précité est destiné à être porté en permanence par l'utilisateur, il importe particulièrement de concevoir un boîtier très compact réduisant au minimum les espaces perdus.

Conformément à l'invention ce boîtier se distingue en ce que le boîtier est essentiellement parallélépipédique et comporte un corps et un couvercle, le corps comportant un fond rectangulaire et des parois latérales perpendiculaires entourant la périphérie du fond de manière à former les côtés du boîtier, à l'exception de deux zones situées en regard l'une de l'autre sur deux côtés opposés, une paroi intermédiaire perpendiculaire étant prévue entre ces deux zones parallèlement auxdits côtés opposés, et en ce que le couvercle se compose d'une plaque rectangulaire munie sur deux bords de deux parois latérales perpendiculaires correspondant auxdites zones lorsque le couvercle est monté sur le corps, et en ce que des moyens de montage sont prévus pour fixer la pompe et son moteur respectivement de part et d'autre de ladite paroi intermédiaire, avec leurs axes perpendiculaires à cette paroi, ladite paroi comportant un orifice pour le passage de la transmission de mouvement entre le moteur et la pompe.

Avantageusement, le corps du boîtier comporte d'autres cloisons séparatrices formant des logements distincts pour les batteries, la plaque électronique de commande, le moteur et une pompe auto-amorçante à pales en caoutchouc.

Un second perfectionnement concerne la réalisation du circuit électronique gérant la détection de liquide et le déclenchement de la pompe.

Conformément à l'invention, le courant d'alimentation des électrodes de détection est une tension alternative, de préférence rectangulaire. Le circuit alimenté en courant continu par les batteries comporte donc un oscillateur à signaux rectangulaires. Cette disposition est particulièrement précieuse pour éviter l'entartrage des électrodes dû à l'électrolyse de l'urine en courant continu.

Selon un autre aspect du second perfectionnement, le circuit électrique comporte également un dispositif manuel de déclenchement de la pompe. Ce dispositif, qui s'ajoute au déclenchement par détection, permet de bipasser ce dernier lors de circonstances particulières : par exemple pour rincer l'ensemble à l'eau, opération quotidienne, il convient que la pompe fonctionne alors que les électrodes ne détectent aucun courant en raison de la résistivité différente entre l'eau et l'urine.

D'autres caractéristiques et avantages de l'invention apparaîtront à la lecture de la description suivante. Il sera fait référence aux dessins annexés sur lesquels :

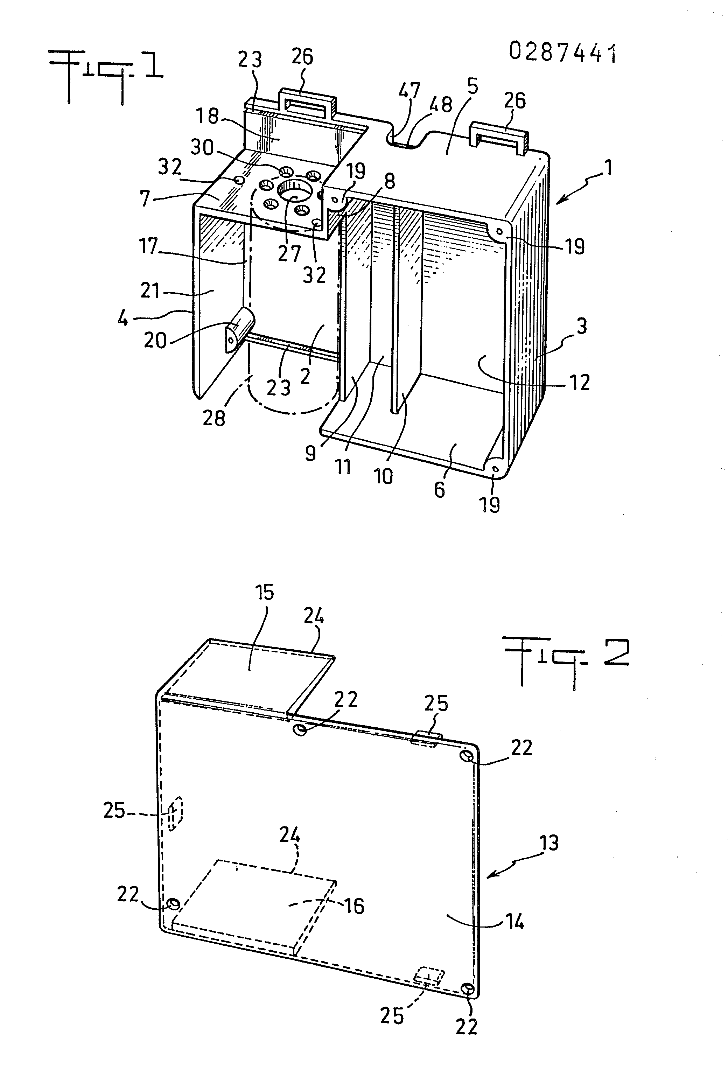

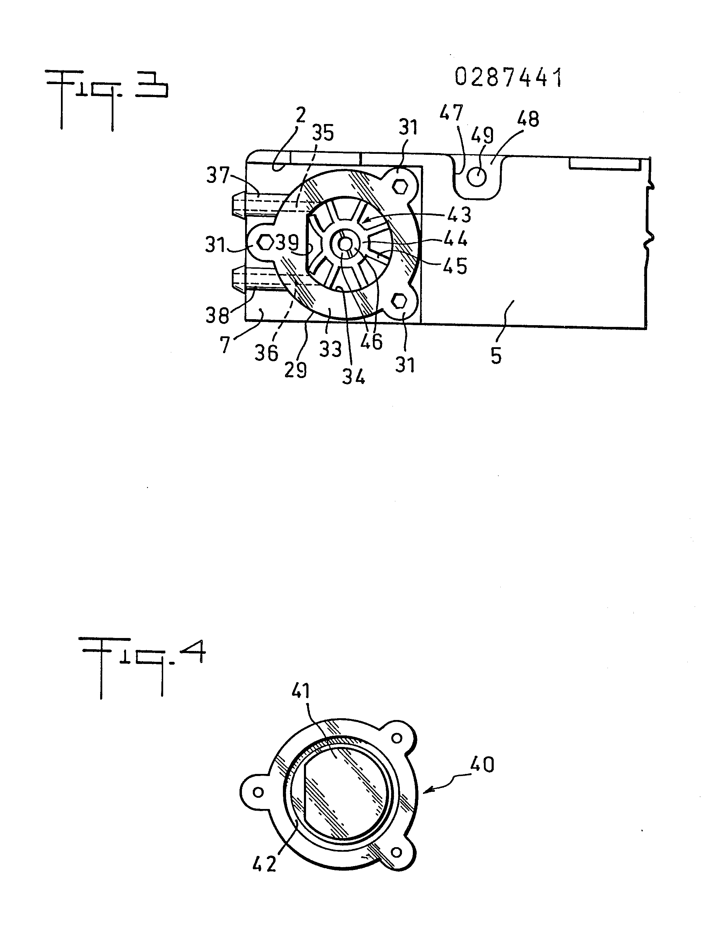

. les figures 1 et 2 sont des vues en perspective du corps et du couvercle de boîtier conforme à l'invention,

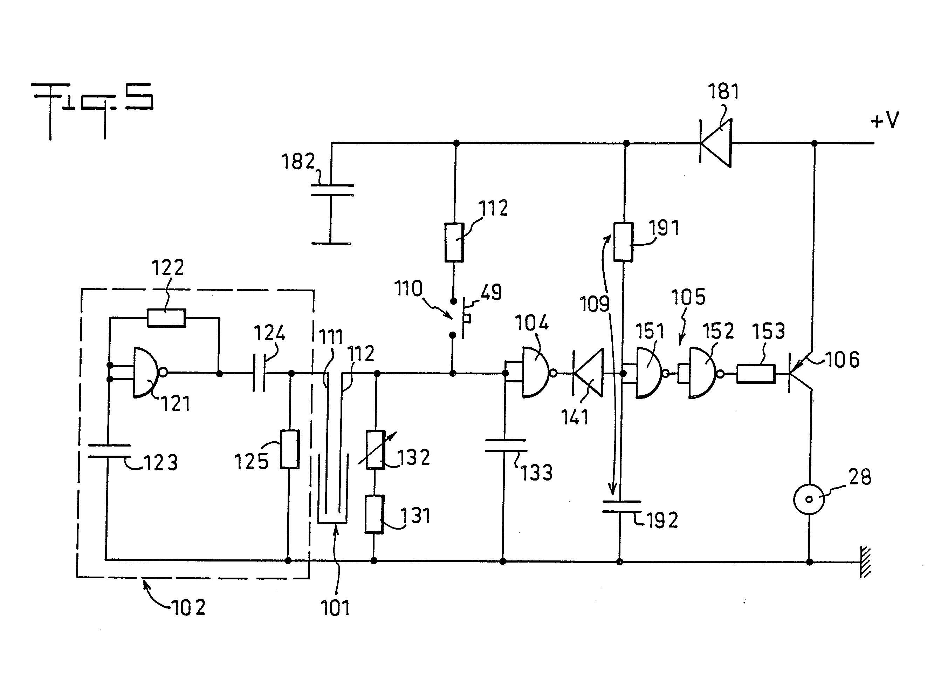

. la figure 3 montre la pompe de l'invention vue de dessus (sans couvercle) en position dans le corps du boîtier,

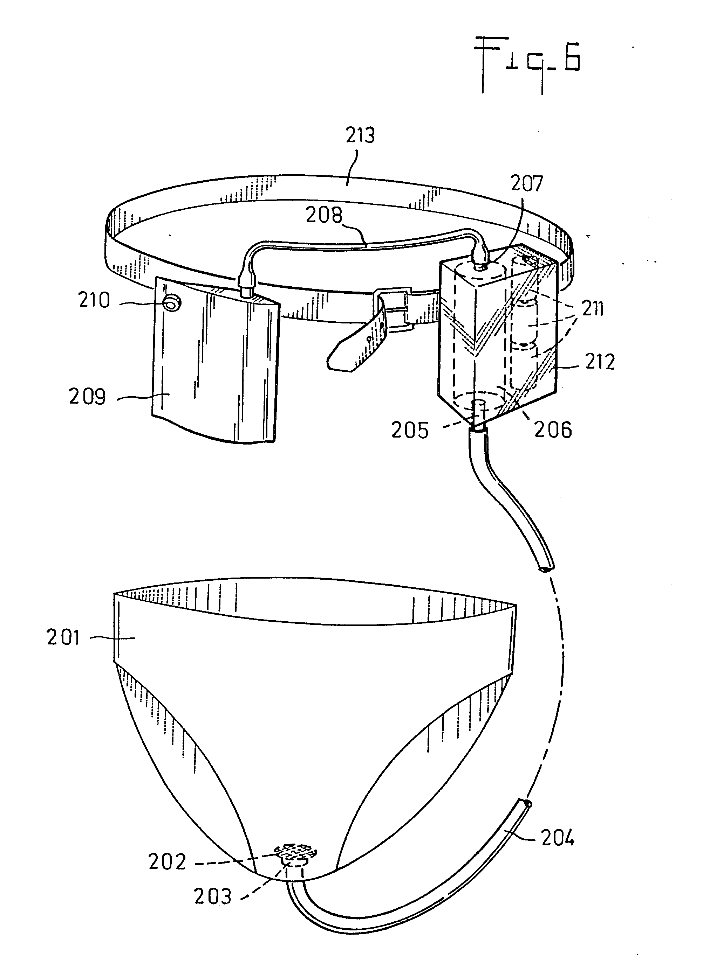

. la figure 4 montre le couvercle de pompe, vue de dessous,

. la figure 5 est un schéma de principe du circuit électronique de commande.

. la figure 6 est une vue schématique du dispositif connu par le document FR-A-2 555 044.

On voit sur la figure 6 un slip ou culotte imperméable comportant au fond de l'entrejambe un prise d'aspiration 202, dotée d'un raccord 203 permettant le branchement d'un tuyau souple 204. Le tuyau souple 204 est branché de l'autre côté sur l'entrée d'aspiration 205 d'une pompe 206, dont la sortie de refoulement 207 est raccordée, par un autre tuyau souple 208, à une poche 209 souple de retenue de liquide pourvue d'une valve de décompression 210. Afin que la poche 209 puisse fonctionner dans toutes les positions du porteur, il est prévu à l'intérieur un flotteur portant sur sa partie émergée l'extrémité d'un tuyau suffisamment long et très souple dont l'autre extrémité est reliée à la valve 210. Ainsi, quelle que soit la position de la poche, le flotteur maintient en dehors du liquide l'extrémité du tuyau qui assure la décompression de la poche.

Au voisinage de la prise d'aspiration 202 se trouvent, non représentées, les électrodes de détection du liquide : elles sont reliées, par un câblage électrique courant en parallèle avec le tuyau souple 204, à un circuit électrique de déclenchement des contacteurs de l'alimentation électrique de la pompe 206. Ce circuit comprend avantageusement un élément temporisateur pour l'arrêt de la pompe. La pompe 206, ledit circuit électrique, ainsi que les batteries 211 nécessaires à leur alimentation, sont contenues dans un boîtier 212.

Le boîtier 212 est retenu à un côté de la ceinture 113, tandis que la poche 209 est retenue de l'autre côté pour équilibrer les charges.

La présente invention vise à perfectionner le boîtier 212 et le circuit électrique.

On voit sur la figure 1 le corps 1 du boîtier, formé d'un fond rectangulaire 2 entouré sur une partie de sa périphérie par des parois latérales perpendiculaires 3,4,5,6. La paroi 3 forme un petit côté du boîtier, et la paroi 4 l'autre petit côté, à l'exception de son bord supérieur laissé dégagé. Les parois 5 et 6 forment les parties en regard des grands côtés, laissant libre une extrémité des grands côtés. Entre ces deux zones laissées libres des grands côtés, une paroi intermédiaire 7 forme un décrochement du petit côté 4 est rattachée au grand côté 5 par une paroi de liaison 8. Une cloison séparatrice 9 prolonge la paroi de liaison 8 et une autre cloison 20 est parallèle à la cloison 9, de manière à former des logements 11 et 12 prévus respectivement pour la plaque électronique de commande, et les batteries d'alimentation électrique. Les cloisons 9 et 10 ne séparent pas le boîtier sur toute sa profondeur, de manière à pouvoir laisser passer entre les différents logements des connexions diverses.

Le couvercle 13 (fig.2) comporte d'une part une plaque rectangulaire 14 dont les dimensions correspondent à celles du fond 2, munie de deux parois latérales 15,16 perpendiculaires à la plaque 14, situées dans les zones laissées libres des grands côtés du corps 1 du boîtier 7. Lorsque le couvercle 13 est monté sur le corps 1, ces parois 15 et 16 sont dans la continuité des parois 5 et 6 respectivement et coopèrent avec le corps du boîtier pour fermer les logements 17 et 18 du moteur et de la pompe.

Quatre chandelles 19,20 placées en angle (19) ou le long d'une paroi (20) sont percées d'un orifice correspondant à des orifices 22 de la plaque 14 pour permettre de fixer le couvercle 13 au corps 1 par des moyens adéquats.

La paroi de fond des logements 17 et 18 comporte, près de la zone libre des grands côtés, une rainure 23 dans laquelle peut s'insérer le bord 24 des parois 15 et 16, profilé ou chanfreiné à cet effet.

Des saillies 25 prévues sur la face interne de la plaque 14 du couvercle permettent de positionner exactement celui-ci sur le corps.

Deux anses 26, venues de matière, sont prévues sur le grand côté supérieur du fond 2 du corps pour servir à la suspension du boîtier.

La paroi 7 comporte un orifice principal 27 pour le passage de la transmission entre le moteur électrique 28 et la pompe 29. Cet orifice est entouré par une couronne de trous fraisés 30 destinés à la fixation par vis du moteur 28. Celui-ci a été auparavant facilement introduit dans le logement 17 et appliqué contre la paroi 7 grâce au fait que le côté inférieur du logement est laissé libre.

Une fois le moteur fixé, on fixe au dessus de la paroi 7 la pompe 29 qu'on maintient par trois vis traversant les chandelles 31 de la pompe pour se visser dans les orifices 32 de la plaque 7.

La pompe plate 29 (fig.3) est constituée d'un corps de pompe 33 auquel se fixe un couvercle 40 (fig.4) par les vis traversant les chandelles 31. Le corps 33 définit une cavité sensiblement cylindrique 34 dont la paroi comporte deux alésages 35,36 communiquant avec des raccordements extérieurs d'aspiration et de refoulement 37,38 dont l'extrémité dépasse légèrement du logement 18 du boîtier, par la paroi non fermée dudit logement. Une portion de paroi plane 39 est prévue entre l'entrée des alésages 35,36.

Le couvercle 40 est conformé pour s'adapter au corps 33 et comporte un plateau central 41 pénétrant légèrement dans la cavité 34 pour la fermer. Une rainure annulaire 42 permet de loger un joint torique assurant l'étanchéïté de la pompe.

Le corps et le couvercle de la pompe sont moulés par injection en une matière plastique dure à bas coefficient de frottement (par exemple résines d'acétal chargée en craie, polycarbonate, PTFE, etc.)

La turbine à pales déformables 43 est moulée en une matière plastique élastique, par exemple en caoutchouc (nitride, EPDM) avantageusement traitée par chloration pour résister aux liquides pompés.

La turbine comporte un anneau central 44 d'où irradient les pales déformables 45. Au centre de l'anneau 44 et d'une pièce avec lui, deux demi-coques cylindriques quasiment jointives 46 permettent la fixation de la turbine sur l'axe du moteur. Ledit axe traverse le fond du corps de pompe 33, où un cuvelage non représenté permet le positionnement d'un joint d'étanchéïté torique ou à lèvre.

Une fois mis en place le moteur 28 et la pompe 29 et les raccordements avec l'électronique de commande et les batteries effectués, le couvercle 13 est mis en place et fixé sur le corps du boîtier 1 qu'il permet de fermer en une seule opération.

L'ensemble des dispositions précédentes permet d'obtenir un agencement très compact du boîtier (7 × 9 × 3,5 cm³) et de plus silencieux grâce à la diminution de toutes les pièces ou espaces de résonnance.

On voit par ailleurs sur les figures 1 et 3 qu'une encoche 47 est formée à l'arrière du corps du boîtier, derrière le logement 11 de la plaque électronique. Cette encoche 47 possède un fond 48, à partir duquel un bouton pressoir 49 fait saillie. Ce bouton commande le fonctionnement manuel de la pompe.

On décrira en référence à la figure 5 un exemple de dispositif électronique, permettant la commande d'une pompe d'assèchement de couche pour incontinent urinaire, conformément à la présente invention.

Le moteur électrique 28 d'entraînement de la pompe est alimenté à partir d'une source d'alimentation +V, par exemple constituée par une batterie d'accumulateurs rechargeable, par l'intermédiaire d'un relais ou, de préférence, comme représenté sur le schéma, par un élément de commutation statique constitué par exemple par un transistor de puissance 106, commandé de façon sélective par un dispositif électronique associé à une sonde 101 disposée dans la couche à assécher.

Un oscillateur 102 à signaux rectangulaires fournit à la sonde 101 une tension alternative rectangulaire à une fréquence par exemple de l'ordre de 125Hz mais qui pourrait être comprise entre environ 1Hz et 10000Hz. La tension alternative rectangulaire délivrée par l'oscillateur 102 avec une puissance comprise entre environ 0,2 et 1 milliwatt permet d'assurer une dépolarisation au niveau de la sonde 101.

L'oscillateur 102 peut être réalisé à partir d'un élément amplificateur inverseur 121 coopérant avec une résistance 122 montée entre l'entrée et la sortie de l'amplificateur 121. L'amplificateur 121 peut être constitué par exemple par une porte NON ET dont les deux entrées sont reliées entre elles. L'oscillateur 102 comprend en outre un premier condensateur 123 connecté entre l'entrée de l'amplificateur 121 et la masse et un second condensateur 124 connecté entre la sortie de l'amplificateur 121 et une électrode 111 de la sonde 101. Une résistance 125 définissant la résistance de sortie du générateur 102 de signaux rectangulaires est connectée entre la sortie de l'oscillateur 102 et la masse.

Un circuit série constitué par une résistance 131 et une résistance ajustable 132 est connecté entre l'électrode 112 de la sonde 101 et la masse. Ce circuit série définit l'impédance de la sonde 101 et la résistance d'entrée de l'amplificateur 104 servant à amplifier les signaux délivrés par la sonde 101. La résistance ajustable 132 permet d'ajuster l'impédance de la sonde 101 et donc la sensibilité de celle-ci en fonction de la plage de résistivité liée au pH urinaire.

Les signaux fournis par la sonde 101 sont intégrés par le condensateur 133 connecté en parallèle sur le circuit série 131,132, puis amplifiés et mis en forme par l'amplificateur 104 qui peut être constitué comme l'amplificateur 121 par une porte NON ET.

La sortie de l'amplificateur 104 est connectée à la cathode d'une diode 141 dont l'anode est reliée à l'entrée d'un second étage d'amplification 105 comprenant deux amplificateurs inverseurs 151,152 montés en série et dont la sortie est reliée par une résistance 153 à la base du transistor de puissance 106. Les amplificateurs inverseurs 151,152 peuvent également être constitués par des portes NON ET.

Le point commun à l'anode de la diode 141 et à l'entrée du second étage d'amplification 105 est relié d'une part au pole positif de la source d'alimentation +V par une résistance 191 et une diode 181, d'autre part à la masse par un condensateur 192.

La résistance 191 et le condensateur 192 constituent un circuit de temporisation 109 qui permet de maintenir sur la base du transistor 106 un signal de commande pendant une durée prédéterminée, par exemple de l'ordre de une seconde, après la fin de l'émission d'un signal par la sonde 101, afin de permettre de vider le reste de la colonne encore rempli de liquide. La durée de la temporisation est ainsi ajustée au volume de la colonne à vider.

Un circuit série 110 constitué par un bouton poussoir 49 et une résistance 112 est connecté entre la cathode de la diode 181 et l'entrée de l'amplificateur 104. Ce circuit série 110 permet d'appliquer artificiellement sur l'entrée de l'amplificateur 104 un signal de tension positive qui permet de faire fonctionner le moteur 28 en marche forcée en cas de rinçage à l'aide d'un liquide tel que de l'eau dont la résistivité est différente de celle de l'urine.

Un condensateur 182 connecté entre la cathode de la diode 181 et la masse permet en outre de découpler l'ensemble du dispositif électronique de commande.

Le fonctionnement du dispositif de commande de la pompe est le suivant.

En cas d'absence de signal en sortie de la sonde 101, l'entrée de l'amplificateur 104 est à un niveau bas et la sortie de celui-ci est à un niveau haut qui empêche la diode 141 d'être conductrice. L'entrée et la sortie du second étage amplificateur 105 sont donc également à un niveau haut, ce qui bloque le transistor 106 et empêche le moteur 28 de fonctionner.

La présence d'un signal en sortie de la sonde 101 fournit après intégration par le condensateur 133 un niveau haut en entrée de l'amplicateur 104 qui produit un niveau bas en sortie de cet amplificateur et rend la diode 141 conductrice déchargeant ainsi le condensateur 192. L'entrée et la sortie du second étage amplificateur 105 sont ainsi à un niveau bas qui rend conducteur le transistor 106 pendant tout le temps où un signal est délivré par la sonde 101, ou par le circuit série 110 lorsque le bouton poussoir 49 est fermé, et pendant le temps supplémentaire nécessaire à la recharge du condensateur 192 qui correspond à la vidange complète de la colonne.

Les électrodes allongées 111 et 112 de la sonde sont placées axialement et sensiblement diamétralement opposées dans une pièce surmoulée entourant l'orifice de l'entrée d'aspiration 205, en saillie légère par rapport au niveau dudit orifice. Cette disposition permet d'éviter un contact électrique intempestif causé par un reste de liquide.

**Revendications**

1. Ensemble pour incontinent comprenant :
a) une culotte réalisée au moins pour sa partie basse en matière imperméable s'adaptant de manière étanche au porteur et comportant en partie basse une zone de puisage (202) à laquelle peut s'adapter un tube souple (204) d'évacuation relié à une poche de retenue (209) au travers d'une pompe (206),
b) un support (213) destiné à être porté en permanence par le porteur, ce support (213) retenant d'une part un boîtier logeant la pompe et une source de courant continu basse-tension, et d'autre part la poche de retenue (209),
c) un circuit électrique temporisé de déclenchement de la pompe, alimenté par la source de courant et comportant des moyens de déclenchement réagissant au liquide disposés près de la zone de puisage (202) et reliés par des fils électriques souples audit circuit,
caractérisé en ce que le boîtier est essentiellement parallélépipédique et comporte un corps (1) et un couvercle (13), le corps (1) comportant un fond rectangulaire (2) et des parois latérales perpendiculaires (3-6) entourant la périphérie du fond de manière à fermer les côtés du boîtier, à l'exception de deux zones situées en regard l'une de l'autre sur deux côtés opposés (5,6), une paroi intermédiaire perpendiculaire (7) étant prévue entre ces deux zones parallèlement auxdits côtés opposés (5,6), en ce que le couvercle (13) se compose d'une plaque rectangulaire (14) munie sur deux bords de deux parois latérales perpendiculaires (15,16) correspondant auxdites zones lorsque le couvercle (13) est monté sur le corps (1), et en ce que des moyens de montage sont prévus pour fixer la pompe (29) et son moteur (28) respectivement de part et d'autre de ladite paroi intermédiaire (7), avec leurs axes perpendiculaires à cette paroi, ladite paroi (7) comportant un orifice (27) pour le passage de la transmission de mouvement entre le moteur (28) et la pompe (29).

2. Ensemble selon la revendication 1, caractérisé en ce que le corps (1) du boîtier comporte d'autres cloisons séparatrices (9,10).

3. Ensemble selon l'une quelconque des revendications 1 ou 2, caractérisé en ce que la pompe (29) est une pompe auto-amorçante à rotor en caoutchouc.

4. Ensemble pour incontinent comprenant :
a) une culotte réalisée au moins pour sa partie basse en matière imperméable s'adaptant de manière étanche au porteur et comportant en partie basse une zone de puisage (202) à laquelle peut s'adapter un tube souple (204) d'évacuation relié à une poche de retenue (209) au travers d'une pompe (206),
b) un support (213) destiné à être porté en permanence par le porteur, ce support (213) retenant d'une part un boîtier logeant la pompe et une source de courant continu basse-tension, et d'autre part la poche de retenue (209),
c) un circuit électrique temporisé de déclenchement de la pompe, alimenté par la source de courant et comportant des moyens de déclenchement réagissant au liquide disposés près de la zone de puisage (202) et reliés par des fils électriques souples audit circuit,
caractérisé en ce que le circuit électrique fournit aux électrodes de détection (101) une tension alternative, de préférence rectangulaire.

5. Ensemble selon la revendication 4, caractérisé en ce que le circuit est alimenté en courant continu et comporte un oscillateur (102) à signaux rectangulaires.

6. Ensemble selon l'une quelconque des revendications 4 ou 5, caractérisé en ce que le circuit électrique comporte en outre un dispositif manuel (49) de déclenchement de la pompe.

Fig.1

0287441

Fig.2

Fig. 3

0287441

Fig. 4

Fig.5

0287441

Fig.6

Office européen des brevets

**RAPPORT DE RECHERCHE EUROPEENNE**

Numero de la demande

EP 88 40 0850

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,A | EP-A-0 148 047 (NIGAY)<br>* En entier * | 1,3 | A 61 F 5/44 |
| Y | | 4-6 | |
| Y | GB-A-2 113 438 (EASTWOOD & SON)<br>* Figures 4A,2,6; revendiations * | 4-6 | |
| A | US-A-4 653 491 (SHIGERU OKADA) | | |
| D,A | FR-A-2 477 881 (KIMURA) | | |
| D,A | FR-A-2 498 447 (INOUE) | | |

**DOMAINES TECHNIQUES RECHERCHES (Int. Cl.4)**

A 61 F

**Le présent rapport a été établi pour toutes les revendications**

| Lieu de la recherche | Date d'achèvement de la recherche | Examinateur |
|---|---|---|
| LA HAYE | 16-06-1988 | STEENBAKKER J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)